# EUROPEAN PATENT SPECIFICATION

(11) **EP 1 475 152 B1**
(45) Date of publication and mention of the grant of the patent: **22.11.2006**
(21) Application number: 03076382.5
(22) Date of filing: 08.05.2003
(51) Int. Cl.: B01L 3/00, G01N 33/543

(54) **Test device**
Test-Vorrichtung
Dispositif pour effectuer un essai

(43) Date of publication of application: 10.11.2004
(73) Proprietor: CEDI Diagnostics B.V., 8219 PH Lelystad (NL)
(72) Inventor: Keizer, Gerrit Dirk, 3845 BV Harderwijk (NL); Schielen, Wilhelmus Joseph Gerardus, 8245 CN Lelystad (NL)
(74) Representative: Prins, Adrianus Willem

(56) References cited:
- EP-A- 0 643 131
- EP-A- 0 940 180
- WO-A-99/35483

## Description

### FIELD OF THE INVENTION

The invention is in the field of biomedical testing and relates to a device for detecting the presence of chemical and/or biological substances in a fluid sample.

### BACKGROUND OF THE INVENTION

Lateral-flow or immunochromatographic tests, methods and devices for the detection of the presence or concentration of chemical and/or biological residues or analytes or in general substances in liquid samples have been developed (see *i.a.* US 6,319,466; US 6,372,516; US6,171,870; US 6,214,629; WO 01/36099; US 6,146,590). An example of such a test device includes the well known early-detection "mid-stream" pregnancy test for detecting human chorionic gonadotropin or hCG.

Particularly in the food safety area it has long been recognized that substance detection, and in particular residue detection should be accurate, inexpensive and easily conducted. Consumers and governments are becoming increasingly aware of the necessity for testing foods for the presence of undesirable residues naturally occurring or otherwise.

For instance, animal husbandry on dairy farms will occasionally necessitate antibiotic treatment of the animals for health reasons. As a result, antibiotic residues may appear in the milk supply. Government agencies have in some cases established legal limits for particular residues in foods, such as antibiotic residues in milk. Only residue levels below the legal limit are considered "safe". It is therefore important that detection methods are, in addition to being inexpensive and easily conducted, accurate and preferably capable of detecting very low amounts of substance, i.e. that they are sensitive.

One problem with the test devices of the prior art is that the quantitative accuracy depends profoundly on sampling accuracy, i.e. on the ability to provide accurate sample volumes to the test. Usually therefore, assay systems require pipetting of the sample into or onto a sample-receiving end of the device, by using either plastic or glass volumetric pipettes. Such fluid transfer devices are preferably not provided when the layman is to use the test, such as in the case of an individual farmer testing his milk for possible contamination. This problem may be solved by such measures as providing the test device with a sample receiver, such as nowadays used in the mid stream pregnancy tests, that comprises a gritted element capable of holding a defined volume of fluid, e.g. 0.5 or 1.0 mL, when briefly contacted with the sample to be tested.

The principle of lateral-flow or immunochromatography on which these devices are based now causes the following problem. Essentially, after intake, the sample fluid is subsequently transported towards, into and past a region of the chromatographic flow-path that comprises a reactant capable of signalling the presence of the tested substance. Transport is by capillary force. Thus, the tested substance or analyte is allowed to establish contact with the signalling reagent only once. As a result, such a prior art test lacks sensitivity or is at least insufficiently sensitive because there is no prolonged contact between the analyte and the signalling reagent or at least only a temporal opportunity for such contact to be established. Also, because there is no equilibrium, such measurements are not quantitative.

Yet another problem with prior art biomedical substance testing devices relates to their relative rapid expiration. The devices of the prior art either cannot be sufficiently closed in an easy manner to completely prevent the entrance of micro-organisms detrimental to the shelf-life of such devices or detrimental to the test itself, or they cannot easily be opened in order to perform the test in a convenient manner.

### SUMMARY OF THE INVENTION

The present invention now provides a test device for determining an analyte in a sample, the device comprising a reagent compartment (1) which is contained in a cylindrical housing (2), which cylindrical housing (2) comprises at least a first open end; and a sample receiver (3) which is also contained in said cylindrical housing (2); and an operating means (4) capable of moving said reagent compartment (1) and/or said sample receiver (3); and wherein the reagent compartment (1) and the sample receiver (3) can be in at least two positions relative to each other, said positions comprising a first position wherein the sample receiver (3) is not in analyte-exchangeable contact with the reagent compartment (1), and a second position wherein the sample receiver (3) is in analyte-exchangeable contact with the reagent compartment (1).

The present invention further provides a method for determining the presence of an analyte in a sample comprising:
a) providing a test device for determining an analyte in a sample, the device comprising a reagent compartment (1) which is contained in a cylindrical housing (2), which cylindrical housing (2) comprises at least a first open end; and a sample receiver (3) which is also contained in said cylindrical housing (2); and an operating means (4) capable of moving said reagent compartment (1) and/or said sample receiver (3); and wherein the reagent compartment (1) and the sample receiver (3) can be in at least two positions relative to each other, said positions comprising a first position wherein the sample receiver (3) is not in analyte-exchangeable contact with the reagent compartment (1), and a second position wherein the sample receiver (3) is in analyte-exchangeable contact with the reagent compartment (1).
b) bringing said sample receiver (3) in contact with the sample;
c) bringing the reagent compartment (1) and the sample receiver (3) in the second position thereby allowing exchange of analyte and/or reagents between the reagent compartment (1) and the sample receiver (3) and allowing the occurrence of a reaction between analyte and reagents and the formation of a detectable product; and
d) determining the presence of the analyte in the liquid sample by visual inspection of the reagent compartment (1) and/or the sample receiver (3) for the presence of the detectable product.

In one aspect, the present invention relates to the use of test device according to the invention for determining an analyte in a sample.

### DESCRIPTION OF THE DRAWINGS

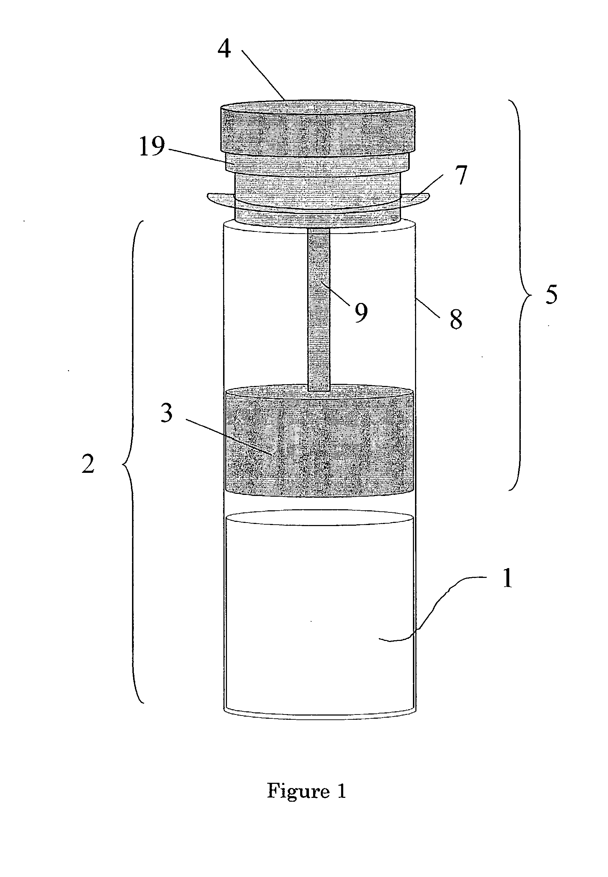

Figure 1 shows a schematic representation of one of the embodiments of the test device of the present invention (see description hereinbelow for details).

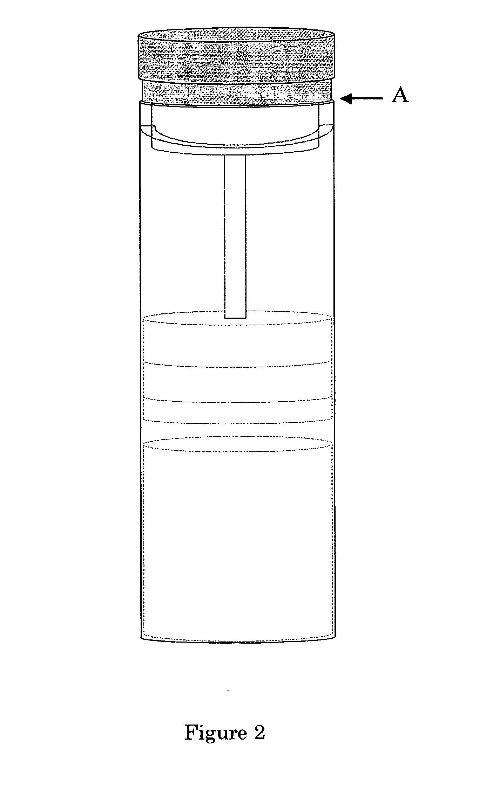

Figure 2 shows a schematic representation of the test device of Figure 1 whereby the reagent compartment (1) and sample receiver (3) are in a first position, whereby the sample taker (5) is located in the cylindrical housing (2), whereby the operating means (4) is at a first hold indicated by position A, meaning that a shoulder or rim (19) thereof is at the edge of the first open end of the cylindrical housing as indicated in Figure 1 and whereby a said operating means (4) is provided with a closing means (7), here in the form of a single flange (see text for details).

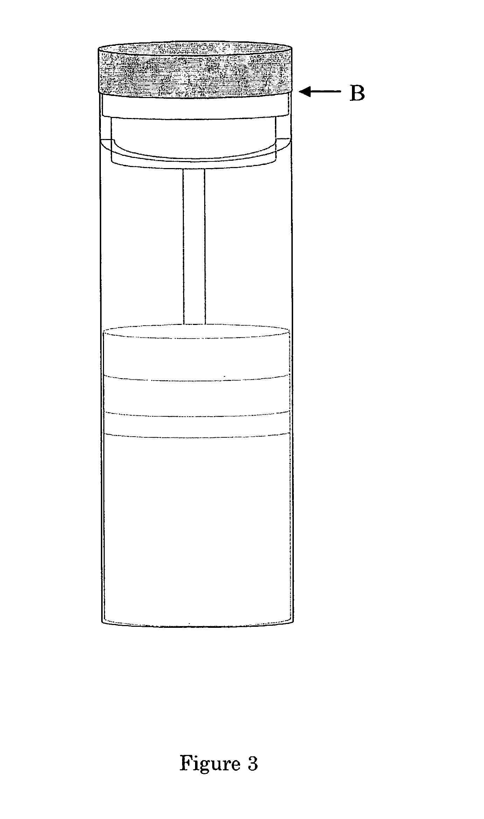

Figure 3 shows a schematic representation of the test device of the present invention whereby the reagent compartment (1) and sample receiver (3) are in a second position, which means that the sample receiver (3) is now in analyte-exchangeable contact with the reagent compartment upon pushing the operating means (4) further into the cylindrical housing (2) towards an end position B as indicated in Figure 1, and whereby the sample receiver (3) and reagent compartment (1) are not in analyte-exchangeable contact (see text for details).

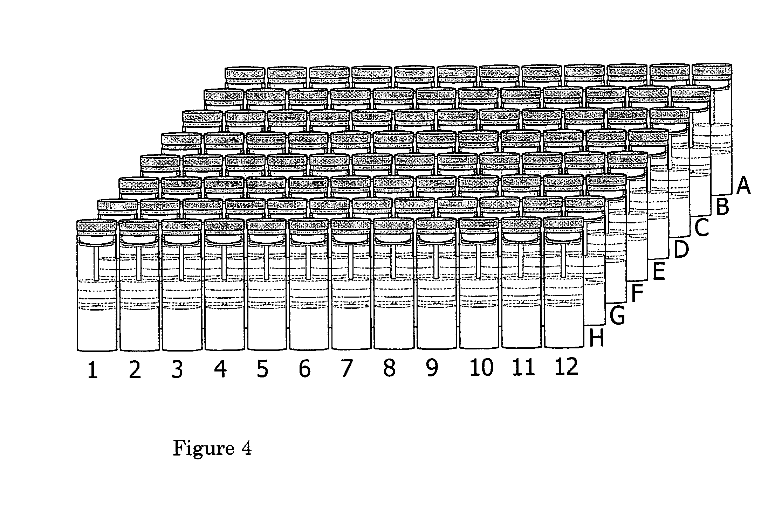

Figure 4 shows an array of test devices in micro-well or 96-well format allowing for mass screening, automation and robotizing.

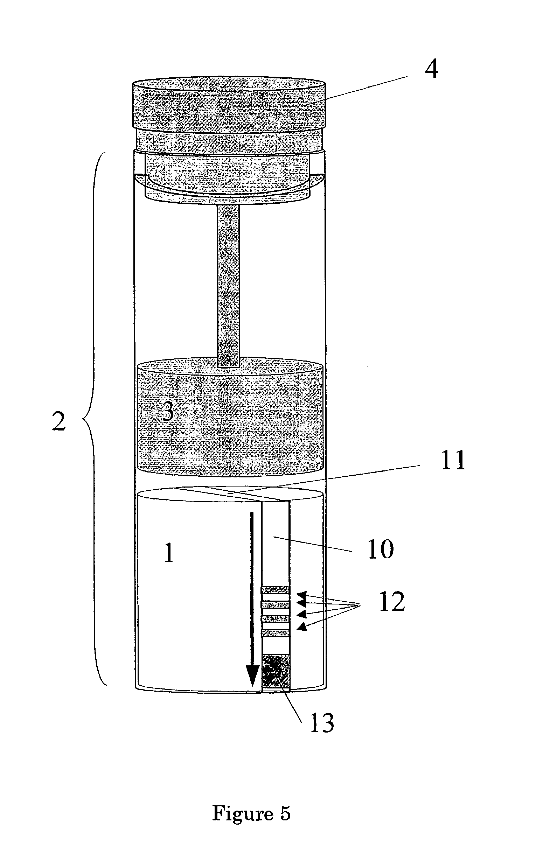

Figure 5 shows a schematic representation of an alternative embodiment of the test device of the present invention wherein the reagent compartment (1) comprises a lateral flow strip.

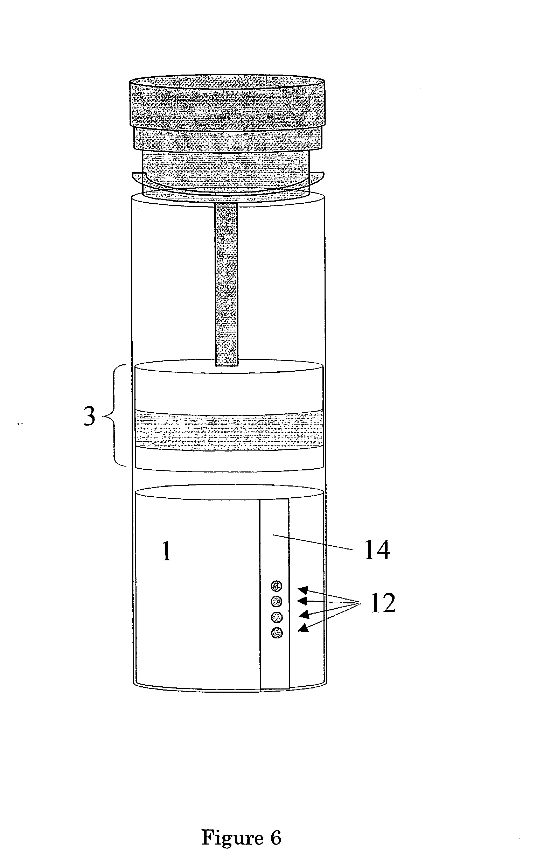

Figure 6 shows a schematic representation of an alternative embodiment of the test device of the present invention wherein the sample receiver (3) comprises multiple layers of materials and wherein the reagent compartment (1) comprises a support structure (14) with test zones (12).

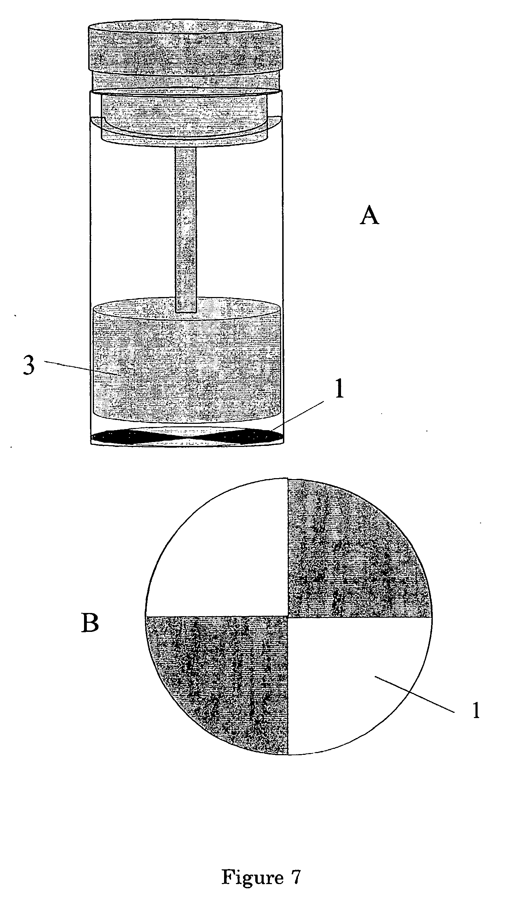

Figure 7 shows a schematic representation of an alternative embodiment of the test device of the present invention wherein the reagent compartment (1) is divided into four distinct compartments. In Figure 7 A, the reagent compartment (1) is positioned on the bottom or base of the cylindrical housing (2). Figure 7B represents a top view in the direction of the bottom of the cylindrical housing (2) of the test device as shown in Figure 7A.

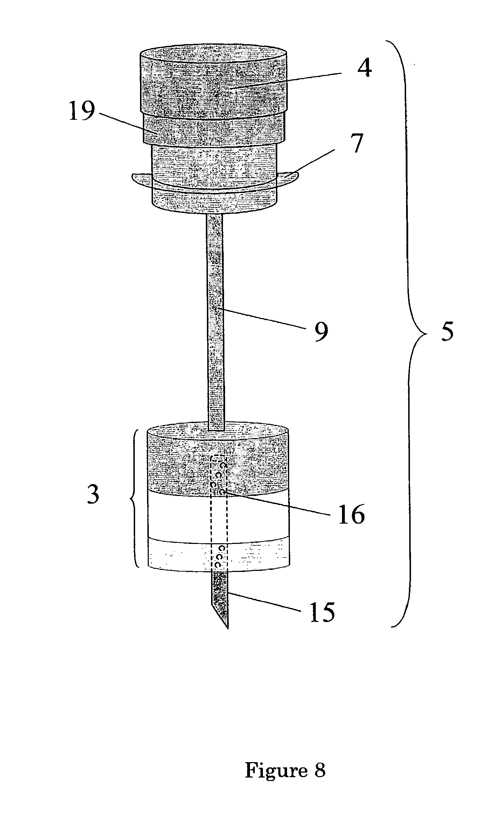

Figure 8 shows a schematic representation of a sample taker (5) wherein the sample receiver (3) is fixedly connected by means of a connecting element (9) to the operating means (4) of the test device of the present invention, wherein the sample receiver (3) comprises multiple layers of materials, and wherein the sample receiver (3) further comprises a probing element (15) and openings (16).

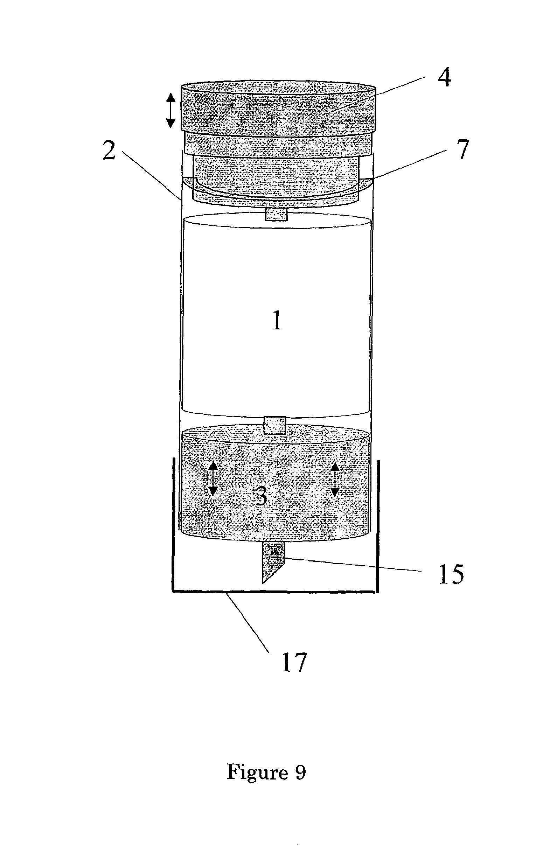

Figure 9 shows a schematic representation of yet another alternative embodiment of the test device of the present invention wherein the sample receiver (3) is positioned between the reagent compartment (1) and the second open end of the cylindrical housing (2), wherein the sample receiver (3) comprises a probing element (15) and wherein the second open end of the cylindrical housing comprises a removable lid (17).

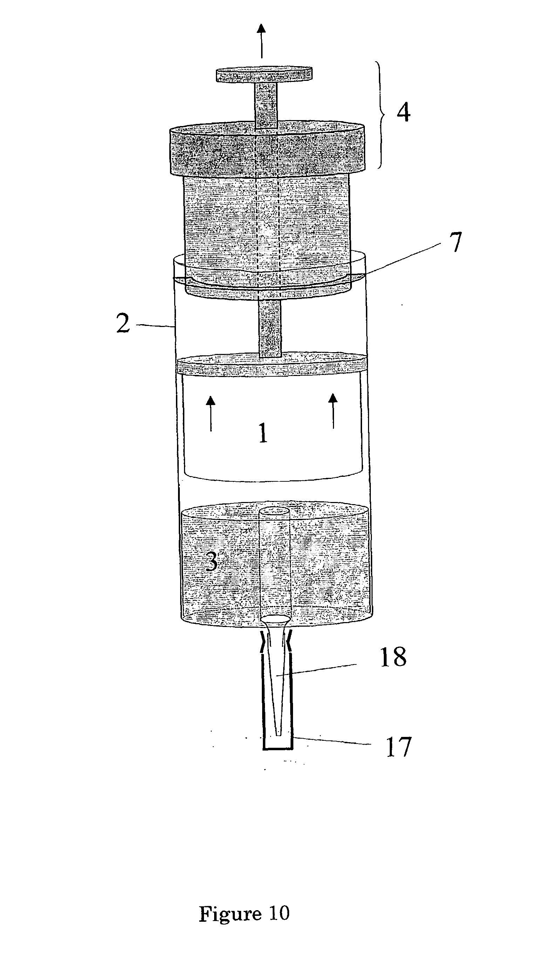

Figure 10 shows a schematic representation of an alternative embodiment of the test device of the present invention wherein the sample receiver is positioned between the reagent compartment (1) and the second open end of the cylindrical housing, wherein the second open end is in the form of a smaller opening and wherein a sample uptake element (18) extends from said opening, which may comprise a removable lid (17).

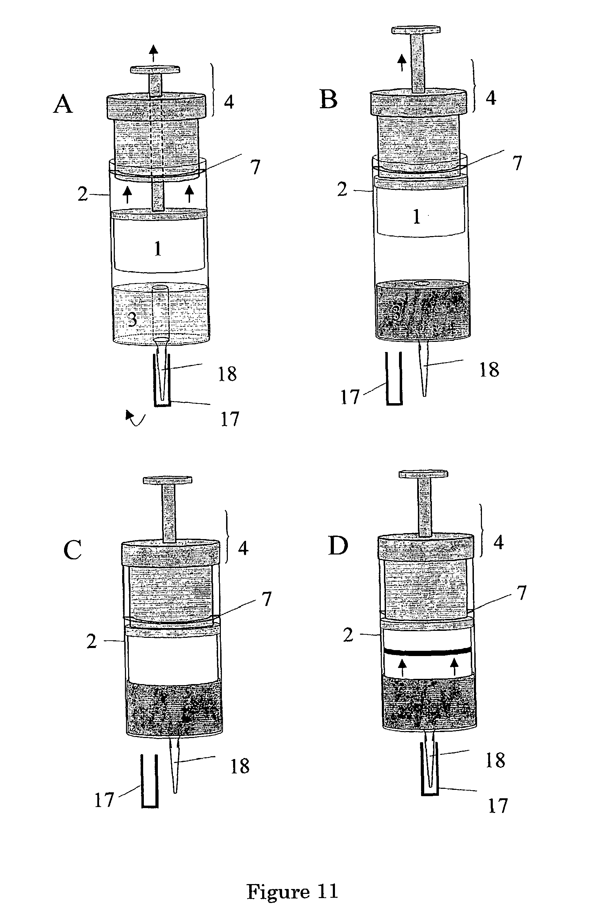

Figure 11 shows the various positions wherein the sample receiver (3) and reagent compartment (1) may be relative to each other in a test device of the invention as shown in Figure 10 during use. The device in storage condition is shown in panel A, upon removal of the removable lid (17) as shown in panel B, the device may be brought in contact with the sample via sample uptake element (18) and a piston comprised in the operating means (4) is pulled outward to create under-pressure in the cylindrical housing (1) as a result of which a (liquid) sample will flow into the sample receiver (3). Pushing the operating means (4) into the cylindrical housing will force excess fluid from the housing through an opening in the sample receiver (3) and will bring the reagent compartment (1) into analyte exchangeable contact with the sample receiver (3) (Panel C). Thereupon the test will start and for instance the analyte will enter the reagent compartment and result in the formation of a detectable product, the presence of which can be determined by visual inspection of the reagent compartment (1) (Panel D).

### DETAILED DESCRIPTION OF THE INVENTION

Many diagnostic test systems rely on the use of pre-determined amounts of sample (eg. whole blood, serum, plasma, urine, saliva, etc.) in order to run the test to its full specifications. The sample volume can influence both test sensitivity as well as specificity. There are many ways of taking exact amounts of samples, however generally a pipette or a capillary tube is used. Therefore (disposable) pipettes, pipette tips, capillaries or other sample takers are often included in the packaging of tests.

The testing device according to the present invention allows the person not normally trained in analytical testing procedures to sample exact volumes. Thereto, the device of the present invention is provided with a sample receiver as an integral part of the test system itself, thereby diminishing the chances of making errors in the sampling process. The detailed description below will elucidate the advantages of its use.

Antibiotic residue tests are extremely important to safeguard foodstuffs, especially milk, from the occurrence of residues. Cows can develop mastitis and most likely will be treated with antibiotics. The milk can only legally enter the food chain after a withholding period, wherein milk containing excess amounts of antibiotic residue is discarded, and which lasts until the milk contains less than the allowed maximum residue limit of the antibiotic used.

Besides the problem of possible allergies in consumers, the presence of residual antibiotic activity in milk should also be prevented for economic reasons. When the milk is used in the preparation of cheese, bacterial starter cultures applied therein may grow at reduced rates or may not grow at all, or the species composition of the complex starter cultures may be altered. All such changes lead to cheeses of unpredictable quality.

Different types of antibiotic residue tests are on the market which can be divided into two major groups
1. Small spectrum fast assay's, as the Delvo-X-Press (DSM, The Netherlands), SNAP (Idexx, USA), Penzym and beta-Star (UCB, Belgium), and MRL and SRL assays (Charm Sciences, USA).
2. Broad spectrum microbial assays, as the Delvotest (DSM, The Netherlands), the P&S ATK assay (Copan; Italy), the BRT-test (AIM; Germany), the Eclipse Test (Spain) and the Charm-Farm test (Charm Sciences, USA).

The first group consists of tests of the ELISA-type or of dry-chemistry devices (dipsticks). Typically a milk sample is taken by the use of a precision pipette or a disposable device and exact amounts of reagents are added to the sample. The tests are based on a tracer-binding inhibition principle and the amount of milk sampled generally influences the sensitivity of the test and is therefore of major importance.

The dry-chemistry devices require incubation at elevated temperatures, usually in an incubator. In some cases the whole test device is heated (SNAP, CHARM MRL), whereas in other cases pre-incubation of conjugate (Beta-Star) is used, after which the test strip is added. All tests require the use of a pipette as sample-taker prior to starting the assay.

The second group, the broad spectrum antibiotic residue tests, have generally two formats. An ELISA plate (96 wells) format or an ampoule format. The ampoule format consists of a sealed (screw cap, aluminium foil) ampoule containing a small amount of agar. The agar contains bacterial spores, a pH or reducer-oxidizer (redox) indicator and occasionally the nutrients, which allow the bacteria to grow. In case the nutrients are initially absent from the agar, they are added from a tablet added to the top of the agar just prior to the application of the milk (Delvotest). After applying a pre-determined amount of milk to the device, the ampoule is heated till 64 °C. The spores germinate to bacteria. As a result of bacterial growth acid and other compounds influencing the redox indicator are produced. Typically, in the absence of antibiotic residues the agar medium changes color after two to three hours of incubation. A sample is defined to be negative if the color changes within a predetermined period of time. The sample is said positive when it does not change color indicating the presence of antibiotics in the sample. The color change at 2/3 of the agar column gives the reading.

These methods suffer from a distinct number of disadvantages. If nutrients are applied to the agar during production, the agar can be a perfect medium for all kinds of undesired micro-organisms to develop, which leads to spoilage of the test. This problem can be circumvented by using aseptical filling methods during production, or by adding the nutrients separately in tablet form just before the start of the assay. The latter approach creates an additional step before the assay can be performed (application of tablet by using a pair of tweezers) but also requires the inclusion of a separate vial in the test kit itself, usually in addition to the pipetting tools.

Moreover if stored improperly, the tablets will attract moisture and will not dissolve properly upon the addition of the sample. Usually this is prevented by addition of a silica sachet to the tablet vials, which adds to the production costs.

The nutrient-containing agar is, upon improper storage of the ampoules, even more vulnerable to infection. In addition, these tests might suffer from reading difficulties, as the milk often tends to leak between the ampoule wall and the agar. As milk is opalescent and tends to absorb the indicators to a certain extent, the appropriate reading of the test result is greatly affected. In case of the use of tablets, the tablet serves as an 'absorbent pad' on top of the agar thereby preventing this problem from occurring. This absorbing effect might however also become disadvantageous. Upon longer incubation or improper coverage of the ampoules during incubation at elevated temperatures, the top of the agar might start to dry-out, thereby potentially generating false positive results.

It is a goal of the present invention to provide for a test device that solves at least some of the problems described hereinabove.

The device according to the present invention has the distinct advantage of providing for a single-step procedure for sample taking and sample testing. Dipping a part of the device into a liquid such as for instance milk provides a pre-determined amount of sample, without the need of using precision pipettes.

During production of the device an absorber or sample receiver (for example an absorbance pad that functions as a sample receiver, that is optionally loaded with a reactant [nutrients and the like] and dried) is fitted or located into a cylindrical housing which housing also contains a reagent compartment. The test device then exists as one self-contained unit. The design is made in such a way that, upon production in a production facility, a sample receiver is brought in a cylindrical housing of the device in a first position whereby no analyte-exchangeable contact between the sample-receiver and the reagent compartment is possible. Upon so linking sample receiver and assay part together, a closed device results. The advantage of such a closed system is that it can thus be stored for prolonged periods of time without the risk of contamination or drying out of the device. An additional advantage therein is that a testing device of the present invention requires no additional packaging to avoid such drying out or contamination. The user will then, just prior to use, merely bring the sample receiver in contact with the sample thereby loading the sample receiver with analyte, which may be done in various ways as is exemplified in various embodiments of the device. Thereupon, the user will bring the sample receiver in analyte-exchangeable contact with the reagent compartment in a second position thereby allowing exchange of analyte and/or reagents between the sample-loaded sample receiver and the reagent compartment. The sample receiver and reagents compartment thus brought together will result in the performance of the assay.

In alternative embodiments of a test device of the present invention, the sample receiver need not be separated from the cylindrical housing for sample taking. In such embodiments the assay involves the removal of a closing lid, entering a probing element or sample uptake element in the sample, allowing the sample receiver to fill with fluid, and bringing the sample receiver and reagent compartment in the second position.

The test device of the present invention may be a singular test device for performing a single test. However, the test device can also employ one or more test zones directed to a variety of tests. Hereinbelow and in the Figures, various embodiments are presented.

The test device is suitable for detecting analytes in any fluid, but preferably body fluids such as, for example, milk, colostrums, urine, (whole) blood, serum, plasma, pleural fluid, gastric fluid, duodenal fluid, intraocular fluid, ascitic fluid, peritoneal fluid, amniotic fluid, synovial fluid, cystic fluid, cerebrospinal fluid, vaginal fluids (including menstrual fluids), semen, sputum, saliva, extracts of faeces or manure, sweat or exudate from lesions.

The test device is also suitable for detecting analytes in cell culture supernatants, water (including potable, cooling tower, waste and process water), extracts of soil or other fluids such as oil.

The sample may either be in the form of a slurry, a suspension, a solution, a macerate, a homogenate, a biopsy tissue sample and the like. Preferably, the sample is a fluid sample or a fluid from a sample.

The test device is particularly useful in the detection of analytes that have to be determined at a certain predetermined level such as toxins, pesticides and antibiotics. The test device is especially useful to detect the maximum residue-analyte limits in a sample. Most of the elements for each test are the same except the chemistries of the detection reagents, which are tailored to the specific analyte detection.

The test device is also useful in the detection of analytes such as nucleotides, peptides, saccharides, or polymers thereof. Also, antibodies, antigens, enzymes, cofactors, hormones, viruses, bacteria, fungi, dioxins, or any other chemical or biological substance for which an, in principle chromogenic, assay can be developed, can be determined by using the present test device. Preferably, visually detectable reaction products are generated through such assays.

The analyte may be present in the sample either in suspended or dissolved form. Preferably, the analyte is present in the test sample in dissolved form.

The test device of the present invention essentially comprises a cylindrical housing (2). The shape of the reagent compartment (1) is in principle cylindrical, the base of which may have any form like square, rectangular, circular etc. with an inner diameter of 1 to 50 mm preferably 2 to 22 mm and a height of 4 to 100 mm preferably 5 to 55 mm. The cylindrical housing (2) is preferably circular cylindrical. The cylindrical housing (2) can be constructed of a flexible or hard material, such as polystyrene, polypropylene, or polyethylene or other thermoplastic resin. Preferably, the cylindrical housing is opaque, more preferably at least locally transparent to allow visual inspection of the reagent compartment (1) and/or the sample receiver (3) for the presence of a detectable product. In one embodiment, a cylindrical housing (2) is employed, such as a one-piece, integral, injection-moulded, all-transparent, plastic material.

In a first embodiment, as illustrated in the Figures 1-3, the cylindrical housing (2) is open at one end so that the sample receiver (3) can be separated from the housing for taking a sample, whereupon the sample receiver (3) containing the test sample may be brought into contact with the reagent compartment (1) comprised within the cylindrical housing (2). The reagent compartment (1) comprised in the cylindrical housing (2) indicates the part or section of the cylindrical housing that functions as a holder or storage place for reagents, more particularly, for reagents involved in the detection of the analyte. Alternatively, and illustrated in other embodiments the reagent compartment (1) may function as inert support carrier for reagents, or as a vessel or receptacle for reaction products or fluid test sample. Although the reagent compartment (1) is defined herein as being located opposite the open end of the cylindrical housing (2), the reader will understand that, in the case of a fluidic reagent compartment, reference is made to the location of the reagents relative to the standard position of the device, which is with the open end of the cylindrical housing upwards.

The reagents in the reagent compartment (1) may either be dry or dissolved, or may suitably be held suspended in a fluid such as a buffer. The reagents in the reagent compartment (1) may also be immobilized in or on a matrix, such as a gel or silica matrix, or any other type of solid matrix that is accessible to fluids and analytes. In case that reagents are dry, no analyte exchangeable contact, as defined herein, is possible between the reagent compartment (1) and the sample receiver (3), and unless they are wetted or moistened, reagents in the dry form will result in a first position as herein defined between the reagent compartment (1) and the sample receiver (3). As stated, the shape of the reagent compartment (1) and/or the solid matrix may differ between various embodiments and is not limited to those herein presented. When the reagents are immobilized in or on a matrix, the diffusion or the flow or in general the movement of the analyte is by such forces as Brown's movement, capillary force, or convection and occurs generally, although not exclusively, from the sample receiver (3) to the reagent compartment (1). Upon contact between analyte and reagent a detectable product as a result of that contact is formed. In this case the reagent compartment (1) functions as a test zone.

Alternatively, the reagents in the reagent compartment (1) may be fully mobile, such as when dissolved in a fluid or buffer. When the reagents are mobile they diffuse or flow or - in general - move by such forces as Brown's movement, capillary force, or convection from the reagent compartment (1) to the sample receiver (3). Thus, contact between analyte and reagent and development of a detectable product as a result of that contact will occur either in the reagent compartment (1) or it may occur in the sample receiver (3) and therefore both reagent compartment (1) and/or sample receiver (3) may function as a test zone.

The reagent compartment (1) may comprise all reagents necessary to allow development of a detectable product upon contact between the analyte-loaded sample receiver (3) and the reagent compartment (1). Alternatively, one or more reagents necessary for the formation of a detectable product may be comprised in the reagent compartment (1) while one or more other reagents necessary for the formation of a detectable product may be comprised in the sample receiver (3). One or more reagents comprised in the sample receiver (3) may for example be comprised therein in dry form and may dissolve and become mobile only upon contact with the sample fluid.

The reagent compartment (1) may comprise such reagents as antibodies, antigens, fluorochromes, quenchers, electrochemical reagents, bioluminescence or chemiluminescence reagents, fungal or bacterial spores, bacteria, viruses, or any substance capable of supporting, mediating, generating or, in general, participating in the formation of a detectable analyte upon contact between one or more reagents and an analyte. In alternative embodiments the reagent compartment (1), comprises indicators, such as redox, pH, etc indicators, for instance provided as indicator layer(s) in the reagent compartment (1). A pH sensitive layer may for instance constitute a color development area in an embodiment of a reagent compartment (1), or a pH indicator or may be included therein. The reagent compartment (1) may also include (combinations of) absorbent materials, mixed together or stacked in a layered arrangement, alternated or divided by (semi)permeable membranes, or different layers may be connected by bridges of chromatographic materials, or the reagent compartment (1) may comprise support structures.

The analyte can suitably be detected in its capacity of a ligand. Broadly, the test device and method of the invention can be used to detect any ligand which has heretofore been assayed using known immunoassay procedures, or known to be detectable by such procedures, using polyclonal or monoclonal antibodies or other proteins comprising binding sites for such ligands. Various specific assay protocols, reagents, and analytes useful in the practice of the invention are known per se, see, e.g., U.S. Pat No. 4,313,734 and U.S. Pat. No. 4,366,241.

The test device of the invention comprises a sample receiver (3). The sample receiver (3) comprises absorbent, sponge-like, bibulous and/or porous material for receiving the fluid test sample. Suitable absorbent materials or sorbents include lightly crosslinked polyacrylate superabsorbents in granular or fibrous form commonly used in disposable diapers such as, for example, Drytech® from Dow Chemical Company, Midland, Mi, USA, Norsocryl® and AquaKeep® of Atofina, Paris, France, and HySorb® of BASF Aktiengesellschaft, Ludwigshafen, Germany. The absorbent material may alternatively be comprised of biopolymeric materials, such as starches or celluloses wherein a the liquid test sample may be taken up under expansion of the sorbent. Such expansion may suitably be used to provide for the occurrence of the second position for testing of the device, i.e. to establish contact between the sample receiver (3) and the reagent compartment (1). Alternative sorbents for taking up test fluids include polyethylene (PE), Polypropylene (PP) Ethylene Vinyl Acetate (EVA), Polystyrene (PS), Epoxy Glass, Phenol Glass or other suitable materials in the form of sintered granulates or sinters. Such materials may be obtained commercially, such as from GenPore, Reading, Pa, USA or Porex Corporation, Fairburn, Ga, USA.

The sample receiver (3) may also include combinations of absorbent materials, mixed together or stacked in a layered arrangement, or alternated or divided by (semi-)permeable membranes, or different layers may be connected by bridges of chromatographic materials or the sample receiver (3) may comprise support structures. Not all materials comprised in the sample receiver (3) are necessarily absorbent. Some materials may be included to provide strength to the sample receiver (3), either as an internal support or as an external (permeable) support layer. The sample receiver (3) may be a rigid, flexible, soft, expandable or any other suitable structure. The skilled person will understand that many arrangements are possible with respect to the sample receiver (3), all of which are within the scope of the present invention. In one embodiment of the test device both nutrients and spores are included in the sample receiver (3). Different layers may be applied in order to prevent inhibition of growth of bacteria by so-called 'natural inhibitors' as lactine and ferritine. A membrane with a low-molecular cut-off level may separate the different layers in the sample receiver (3).

The sample receiver (3) may further comprise reagents, preferably, such reagents as are necessary for completion of the intended reaction that are not as yet comprised in the reagent compartment (1).

Since it is essential that the sample receiver (3) and the reagent compartment (1) can be in direct contact, the structure, shape and dimension of the sample receiver (3) will bear relation to the structure, shape and dimension of the reagent compartment (1). For example, when the reagent compartment (1) comprises a fluid, the sample receiver (3) may simply be submerged therein, or may contact the surface thereof. Alternatively, when the reagent compartment (1) comprises a gel or solid, the sample receiver (3) may contact the surface thereof. As a result, the shape and dimension of the sample receiver (3) may be determined by the shape and structure of the reagent compartment (1), but need not necessarily be equal in shape or dimension thereof.

Also the structure, shape and dimension of the sample receiver (3) will bear relation to the structure, shape and dimension of the cylindrical housing (2), but is not essentially limited thereby as long as the sample receiver (3) can be contained in the cylindrical housing (2).

The test device of the invention further comprises an operating means (4) capable of moving the reagent compartment (1) and/or the sample receiver (3). Said operating means may also ensure closing of the first open end of the cylindrical housing, preferably an air-tight closure of the cylindrical housing and may therefore suitably comprise a closing means (7), for instance in the form of a flexible sealing flange that becomes wedged between a portion of the operating means (4) that enters the cylindrical housing (2) and the wall (8) of the cylindrical housing (2), preferably both in the first and second position. The air-tight closure of the cylindrical housing prevents the contents to attract moisture or to dry-out or to become microbially contaminated. A closing means (7), for instance in the form of a flexible sealing flange is suitably prepared from a compound such as polypropylene. The flexible sealing flange may be a single flange, but may also comprise multiple flange layers. Alternatively, multiple flanges may be provided for even better closure.

The operating means (4) may further have a holding or gripping function, *i.e.* when the sample receiver (3) is fixedly connected to the operating means so as to form a sample taker (5), to facilitate the taking of samples and manipulating the sample taker (5) separately from reagent compartment in the cylindrical housing.

During production and storage, the sample receiver (3) is positioned in the cylindrical housing in such a way that no analyte-exchangeable contact between the sample receiver (3) and the reagent compartment (1) in a first position is possible (Figure 2). In this position, the open end of the cylindrical housing (2) is closed by the operating means (4) to avoid drying out or contamination of the reagents.

The use of the device includes contacting the sample receiver (3) with the sample, and then contacting the sample receiver (3) with the reagent compartment (1) in a second position (Figure 3). In this position, the open end of the cylindrical housing (2) is still closed by the operating means (4) but the sample receiver (3) is in analyte-exchangeable contact with the reagent compartment (1).

In many embodiments, such as those presented in the Figures 1-8, the first step in the functional use of the device for the purpose of testing a test sample for the presence of an analyte, will involve the separation of the sample receiver (3) from the cylindrical housing (2). The sample receiver (3) is then contacted with the test sample so that a quantity of said sample is applied thereto. Preferably, the sample receiver (3) is contacted with the test sample to become fully saturated with a liquid sample, so that a standard amount of sample is applied to the sample receiver (3) and separate sampling procedures yield repeatable sample volumes.

The sample receiver (3) may be connected via a connecting element (9) to the operating means (4) and can be brought into contact with the reagent compartment (1) after the sample has been absorbed. (Push or screw to the 'second position' [B in Figure 1; Figure 3]). Both methods which have been used in the prior art can be carried out using this system. To perform the method using the nutrient tablet, the sample receiver (3) can during production of the test device be soaked into a nutrient solution and then dried, whereby during the assay it serves as a sample and nutrient delivering system. To perform the method using the incorporated nutrients, the sample receiver (3) is only used as sample applicator. However, in contrast to the prior art embodiments as the sample receiver (3) comes into contact with the total surface of the agar-solidified reagents compartment, the risk of contamination with other micro-organisms as a result of improper sealing or storage is strongly reduced.

Also, because the sample receiver (3) serves as an absorbent layer, milk will not tend to leak between the agar and the wall of the cylindrical housing (2) and therefore the reading of the assay is facilitated.

After bringing the loaded sample receiver (3) in analyte exchangeable contact with the reagent compartment (1), some time is allowed to pass during which exchange of analyte and/or reagents between the reagent compartment (1) and the sample receiver (3) is permitted, this time is referred to as the reaction period. This exchange of analyte and/or reagents results in contact between the analyte and one or more reagents and the occurrence of a reaction therebetween to form a detectable product.

It is not essential that the sample receiver (3) is in analyte exchangeable contact with the reagent compartment (1) during the whole of the reaction period. Occasionally, for instance, the device may be turned, so that the dissolved reagents in the reagent compartment (1) are brought into renewed contact with the sample receiver (3).

The term "analyte exchangeable contact" as used herein, refers to a period of contact between the sample receiver (3) and the reagent compartment (1) which is sufficient to allow at least some of the analyte contained in the sample receiver (3) to encounter the reagents of the reagent compartment (1) and which occurs under conditions wherein exchange of analyte and/or reagents between the reagent compartment (1) and the sample receiver (3) is possible. Such conditions thus preferably comprise conditions wherein diffusion can occur, such as moist or wet conditions.

When sufficient time has elapsed, the outcome of the assay is determined by visual inspection of the reagent compartment (1) and/or the sample receiver (3), wherein the presence of the analyte will be indicated by the presence of a detectable product. Such a detectable product may be a coloration of (a part of) the reagent compartment (1) or sample receiver (3), formation of cloudiness therein, or the generation of luminescent or fluorescent signals, depending on the type of reaction chosen.

In Figure 5, a schematic presentation of an alternative embodiment of the test device of the present invention is shown, wherein the reagent compartment (1) comprises a lateral flow strip (10). This lateral flow strip (10) extends from the top of reagent compartment (1) either in one or in more directions to the wall (8) of the cylindrical housing (2) and extends along the wall in a direction towards the bottom or closed end of the cylindrical housing (2). The lateral flow strip (10) may be in contact with the top of reagent compartment (1) and the portion of the lateral flow strip (11) that is positioned on the top of reagent compartment (1) is preferably positioned such that at least an analyte-exchanging contact between the lateral flow strip (10) and the sample receiver (3) is possible. The reagent compartment (1) herein also comprises a support element for holding the lateral flow strip (10). As a consequence the movement of the analyte is restricted to capillary movement through the lateral flow strip (10) in a flow path the direction of which is indicated by the arrow in Figure 5. In such an embodiment, the necessary reagents may for instance be comprised in (part of) the lateral flow strip (10) or in the test zones (12). These test zones (12) represent binding zones or in general reaction zones for the analyte and may optionally comprise one or more control zones for evaluation of the reaction. The reagent compartment (1) may comprise absorbent or bibulous material for drawing the fluid (from the) test sample from the sample receiver (3), into the portion (11) of the lateral flow strip, through the lateral flow strip (10) in one or in more directions towards the wall and further towards the bottom of the cylindrical housing (2), into the reagent compartment (1). The analyte that is included in the fluid (from the) test sample is thereby passed by and through the various test zones (12). In order to prevent direct diffusion of the analyte into an absorbent material of the reagent compartment (1), the lateral flow strip may for instance comprise a non-permeable backing on one side, which side is then faced towards and is in contact with the reagent compartment (1), while the other side is allowed to make analyte-exchanging contact with the sample receiver (3) and draw analyte therefrom. Additionally, those parts of the top of reagent compartment (1) that are not covered by the lateral flow strip may then be covered by a non-permeable material that prevents analyte from directly entering the absorbent material comprised in the reagent compartment (1). Some part of the lateral flow strip (10) that extends along the wall of the cylindrical housing (2) in the direction of the bottom of the cylindrical housing (2) is preferably able to make analyte-exchanging contact with the absorbent or bibulous material comprised in the reagent compartment (1) at a position (13) which lies beyond the test zones (12) in the direction of the flow path. Such analyte-exchanging contact may for instance be achieved by removing the non-permeable backing from the lateral flow strip (10) in position (13) so as to allow entering of test fluid from the lateral flow strip (10) into the absorbent material of the reagent compartment (1). A test device in an embodiment as shown in Figure 5 is capable of measuring multiple analytes simultaneously since various test zones (12) may be provided. Furthermore, multiple lateral flow strips (10) may be comprised in such a test device, including those which are commercially available.

Another embodiment of a test device of the present invention is shown in Figure 6. The reagent compartment (1) herein comprises reagents contained for instance in a (liquid) reaction buffer. The reagent compartment (1) is preferably positioned such that an analyte-exchanging contact between the reagent compartment (1) and the sample receiver (3) is possible. The analyte(s) included in the fluid (from the) test sample then diffuse from the sample receiver (3) into the reagent compartment (1). The reagent compartment further comprises a support structure (14) which support structure comprises test zones (12) that represent binding zones or in general reaction zones for the analyte. The test zones (12) may optionally comprise one or more control zones for evaluation of the reaction. In such an embodiment, the necessary reagents may thus be comprised in the reagent compartment (1) and in the test zones (12). The support structure (14), comprising the test zones (12), is preferably positioned along the wall (8) of the cylindrical housing (2), may be fixed thereto by any means known in the art, may take any suitable shape, and may cover any suitable part of said wall (8). Even multiple support structures (14) may thus be affixed to the wall of cylindrical housing (2). The test zones (12) on the support structure (14) are positioned such that analyte- and reagent-exchanging contact between the analytes and reagents comprised in the reagent compartment (1) and the test zones (12) is possible. Such an embodiment differs thus from that presented in Figure 5 in that in the present embodiment the analyte(s) are in continuous contact with the reagents in the reagent compartment (1) and in the test zones (12). Upon prolonged contact, reaction equilibrium may take place. The advantage of this embodiment thus pertains to its high sensitivity, and capability for quantitative measurements.

In yet another alternative embodiment, the reagent compartment (1) may be reduced to a flat surface on the bottom of the cylindrical housing (2). Such a flat surface may for instance be comprised of a reagent containing strip, such as a pH testing paper or any other reagent strip. Upon contact with sample receiver (3) and said flat reagent compartment (1) the reaction is started.

In order to perform multiple tests simultaneously in one test device, the reagent compartment (1) in any embodiment of a test device of the present invention, may be divided into two or more different compartments, each of which compartments may comprise the same or different reagents suitable for one or more specific reactions. Figure 7B represents a top view in the direction of the bottom of the cylindrical housing as shown in Figure 7A wherein a (flat) reagent compartment (1) is represented that is divided into four distinct compartments, each capable of supporting a different assay. Preferably, an isolated part of the sample receiver (3) is in analyte exchangeable contact with a likewise isolated part of the multi-compartmental reagent compartment (1) and preferably therefore the positioning of the sample receiver (3) *vis a vis* the reagent compartment (1) is fixed. Thereto, the cylindrical housing may for instance be provided with a notch and the sample receiver (3) with a corresponding depression so that their orientation relative to each other is fixed. Apart from various embodiments in the design and functionality of the reagent compartment (1) of a test device of the present invention, the sample receiver (3) and operating means (4) may also be provided in various embodiments. In Figure 8, a schematic presentation of an alternative embodiment of the sample receiver (3) of the test device of the present invention is shown. Herein, the sample receiver (3) is provided with probing element (15). Said probing element (15) may be piercing, incisive, stabbing or cutting, and may be provided in the form of a canula, a (hollow) needle, a blade, a scalpel or the like. The probing element is provided for the purpose of sampling, such as for instance the taking of blood samples or biopsies from humans or animals. The purpose of a hollow needle, as drawn in Figure 8, would be to penetrate a skin and to draw blood into the sample receiver (3), *e.g*. through capillary action. Thereto, the hollow needle may be provided with small openings (17) through which the blood or other sampling fluid may enter into one or more regions of the sample receiver (3). The purpose of a scalpel as a probing element (15) would be to create a skin incision or wound, the size of which would depend on the size of the scalpel. By pushing the test device as shown in Figure 8 on a human or animal subject would drive the scalpel through the skin, thus creating a bleeding wound. By keeping the test device and in particular the sample receiver (3) forced against the skin, blood emerging from that wound would be absorbed into the sample receiver (3) and a blood sample is obtained. The probing element (15) may also briefly emerge from the sample receiver (3) upon sampling and then withdraw therein, such as for instance by spring action activation. Spring action activated needles are well known in *e.g.* skin punctuators for blood glucose testing. The construction for such a mechanism may be provided within the sample receiver (3) and/or other part of the test device. The probing element (15) may for instance be part of the connecting element (9) between the sample receiver (3) and the operating means (4). It will however be clear to the skilled artisan that the probing element (15) may be integrated or fastened to the test device in any suitable way.

Yet further alternative embodiments are possible in a test device of the present invention. Figure 9 shows an embodiment of a test device of the invention wherein the sample receiver (3) is located between the reagent compartment (1) and the second open end. In this case the cylindrical housing (2) is open, or at least has openings, at both ends. In the illustrated embodiment, the connecting element (9) passes through the reagent compartment (1). As a result, the operating means (4) and sample receiver (3) are at opposite sites of the reagent compartment (1). During storage, the second open end of the cylindrical housing (2) is closed by means of a removable lid (17) and the sample receiver (3) is not in analyte-exchangeable contact with the reagent compartment (1). Prior to sampling, the removable lid (17) is removed. Sampling then involves holding the test device against an object for testing, whereby the sample receiver (3) is in contact with the object for testing thereby retrieving a sample from the object, and pulling the sample receiver (3) back into the cylindrical housing (2) in a position whereby the sample receiver (3) comes in analyte-exchangeable contact with the reagent compartment (1). The advantage of such an embodiment is that sample taking and testing can be performed with extreme ease and the sample receiver (3) does not needs to be separated from the housing (2). Especially in the case of for instance blood testing of pigs, a procedure that uses such a test device minimizes stress to the animals. In particular, in embodiments wherein a probing element (15) is comprised in the second element, the procedure involves only a single action of pushing, holding, retrieving and testing.

In stead of providing the sample receiver (3) with a probing element (15), the cylindrical housing (2) may also comprise a probing element. A very suitable probing element in such an embodiment includes a sample uptake element (18), such as a pipette tip or a needle. Such an embodiment is depicted in Figures 10 and 11, wherein the second opening of the cylindrical housing (2) is provided with a sample uptake element (18) which extends from said second open end of the cylindrical housing (2) in the form of a pipette tip. The pipette tip may be covered by a removable lid (17). Again, the operating means (4) and sample receiver (3) are at opposite sites of the reagent compartment (1). During storage, the sample receiver (3) is not in analyte-exchangeable contact with the reagent compartment (1). Prior to sampling, the removable lid (17) is removed (Figure 11, panel B). Sampling then involves pushing the sample uptake element (18) in an object for testing or a sample, allowing sample to be retrieved by the sample receiver (3), for instance by pulling a piston fixedly connected to the reagent compartment to create an area of reduced pressure behind the sample receiver (3), and then pushing the entire operating means (4) into the cylindrical housing (2) to force the reagent compartment into analyte exchangeable contact with the sample receiver (3).

Such embodiments wherein a sample is drawn into the test device by positive displacement or air-displacement are very suitable for obtaining fixed volumes of samples and may for instance be in the form and mechanism as used in a manual or electronic single or multichannel pipettes, such as air-displacement pipettes or positive displacement pipettes as provided by Gilson or Eppendorf, wherein parts such as a piston, seal, O-ring and tip holder are integrated.

A device of the present invention may be used for testing milk as described herein or for testing blood or tissue for the presence of certain agents, such as BSE or infections such as viral infections or bacterial or fungal infections.

The invention will be further understood from the following nonlimiting examples.

### EXAMPLES

### Example 1

A commercially available antibiotic residue screening test in ampoule format (CH ATK) was obtained from Copan (Brescia, Italy). This test consists of pipettes for milk sampling and test tubes comprising spores of *Bacillus stearothermophilus* var. *calidolactis* trapped in an agar gel matrix, which gel matrix further contains nutrients and a (purple) pH indicator. The test tubes are sealed with aluminium foil. In order to start the test the foil is punctured with the pipette or pipette tip, and a milk sample is applied to the agar surface. Subsequently the test tubes are incubated for two hours and 30 minutes at 64 ±0.5 °C.

In case the tested milk does not contain antibiotic residues, the color of the reaction medium (reaction zone) changes form purple to yellow as a result of the acid produced by the bacteria growing in the reaction zone. (*i.e.* the spores germinate and the bacteria start to grow).

In case the milk does contain certain amounts of antibiotic residues, the bacterial growth is inhibited. As a result the reaction zone does not change color after the incubation period as no acid is produced due to the inability of the bacteria to grow.

The above described CH ATK test was used as control in the following example:
Using a pipette, 150 µl of antibiotic-free milk was placed onto the reaction zone of three test tubes (triplicate). Using the same application method, three other test tubes were loaded with 150 µl of milk containing 10 ppb of penicillin G.

The CH ATK test tubes served as cylindrical housings and reagent compartments of a test device according to the present invention and a sample receiver and an operating means according to the present invention were prepared and used in conjunction therewith.

Different types of the sample receivers connected to operating means according to the present invention were produced. Onto plastic rods that fitted into the test tubes, different types of Whatman filter paper were applied. The Whatman filter paper served as sample receiver. The paper discs had the same diameter as the inner diameter of the CH ATK test tubes at the level of the agar surface. The thickness of the sample receiver was adapted in such a way that it could absorb 140-160 µl of milk. The thickness was experimentally determined.

The sample receivers with the different filter papers were dipped or soaked in various milk samples for 5 seconds to absorb the required amount of test fluid. For each type of filter, three sample receivers were soaked in antibiotic free milk and three were soaked in milk containing 10 ppb of penicillin G. The sample receivers, were taken out of the milk, inserted into the test tubes so that the sample receivers were in analyte-exchangeable contact with the agar surface of the CH ATK test.

The test tubes were placed in an incubator and heated at 64 °C for 2 hours and 30 minutes. The pH indicator in the reagent compartment of the test tubes containing antibiotic-free milk changed color from purple to yellow, whereas the test tubes with the 10 ppb of penicillin G containing test milk did not change color, not even after prolonged incubation.

A titration curve was made wherein different amounts of penicillin G were added to a test-milk. The results showed that the sensitivity of a test whereby the milk was applied by using sample receiver according to the present invention was similar to the original CH ATK test wherein the milk samples were applied by a pipette. All tests were able to detect penicillin G additions of 3 ppb, and were not able to detect penicillin G additions lower than or equal to 1 ppb.

### Example 2

In order to show an additional advantage of the use of a test device of the present invention, the experiment of Example 1 was repeated with test tubes of the CH ATK test that were forcefully brought in contact with a hard surface (table top) in such a way that the agar gel matrix came lose from the wall of the test tube. As a result, an air-filled space between the wall of the test-tube and the agar gel matrix was created.

The placing of 150 µl of milk onto the agar gel matrix by using a pipette resulted in milk entering the space between the agar gel matrix and the test tube wall and development of an opalescent zone therebetween. Upon incubation at 64 °C, this zone disturbed a clear reading of the device, especially at sub-optimal concentrations of penicillin G. The sensitivity of the test was reduced to a value between 2 and 5 ppb of penicillin G. A reliable determination of the exact sensitivity could not be made as a result of difficulties in reading due to the opalescent, purple/yellow zone between the vessel wall and the reaction zone.

Using a sample receiver of filter paper with low capillary capacity had a less intense although similar effect. However, less milk was detectable between the test tube wall and the agar, which resulted in a better readability of the color change.

Using filter paper with smaller cavities (i.e. a high capillary forces) showed either no traces of milk or a minimal amount of milk between the test-tube wall and the agar. The reading of the test was not affected and the sensitivity was between 1 and 3 ppb.

Using disc-shaped sample receivers with a diameter slightly smaller than the inner diameter of the test tube revealed the best results. No milk could be detected between the test tube wall and the agar, and the sensitivity of the test was not affected.

### Example 3

A commercially available antibiotic residue screening test in test-tube format (Delvotest® SP ; US 3,941,658) was obtained from DSM Food Specialties (Delft, The Netherlands). This test is similar to the CH ATK test described above in that it consists of single test ampoules or test tubes containing a solid and buffered agar medium which contains a pH indicator (bromocresol purple) and spores of *Bacillus stearothermophilus.* The test tubes are sealed by aluminium foil. In order to start the test the foil is punctured by a pipette or pipette tip, a nutrient tablet is placed on top of the agar by use of a pair of tweezers or a dedicated dispenser and a milk sample is applied by using a syringe with pipette tips.

The above described Delvotest® SP test was used as control in the following example: A nutrient tablet was placed on the agar medium of six test tubes. Using the syringe and the pipette tips, 100 µl of antibiotic-free milk was applied to the three test tubes thus prepared (triplicates). Using the same method, three other test tubes were loaded with 100 µl of milk containing 10 ppb of penicillin G. During incubation the test tubes were covered with an adhesive foil in order to prevent drying out.

The Delvotest® SP test tubes served as cylindrical housing and reagent compartment of a test device according to the present invention and a sample receiver and a operating means according to the present invention were prepared and used in conjunction therewith.

Different types of sample receivers according to the present invention were produced. Onto plastic rods that fitted into the test tubes, different types of Whatman filter paper were applied. The Whatman filter paper served as sample receiver. The paper discs had the same diameter as the inner diameter of the Delvotest® SP test tubes at the level of the agar surface. The thickness of the sample receiving portion was adapted in such a way that it could absorb 90-110 µl of milk. The thickness was experimentally determined.

The sample receiver was loaded with nutrient solution and dried. The nutrient solution was prepared by diluting nutrient tablets from the Delvotest® SP test into distilled water. An equivalent of 100 µl of distilled water was used per tablet. Each sample receiving portion was loaded with 100 µl of this solution prior to drying.

The sample receivers with the different nutrient-loaded filter papers were dipped or soaked in various milk samples for 5 seconds to absorb the required amount of test fluid. For each type of filter, three sample receivers were soaked in antibiotic free milk and three were soaked in milk containing 10 ppb of penicillin G. The sample receivers were taken out of the milk, inserted into the test tubes so that the sample receiver thereof was in analyte-exchangeable contact with the agar surface of the Delvotest® SP agar surface.

The control tests (original Delvotest® SP) and the modified tests (with sample receivers according to the present invention) were placed in an incubator and heated at 64 °C for 2 hours and 45 minutes. The pH indicator in the reagent compartment of the test tubes containing antibiotic-free milk changed color from purple to yellow, whereas the test tubes with the 10 ppb of penicillin G containing test milk did not change color, not even after prolonged incubation.

A titration curve was made wherein different amounts of penicillin G were added to a test-milk. The results showed that the sensitivity of a test whereby the milk was applied by using a sample receiver according to the present invention was similar to the original Delvotest® SP test wherein the milk samples were applied by a pipette to a nutrient tablet. All tests were able to detect penicillin G additions of 2 ppb, and none were able to detect penicillin G additions lower than 1 ppb.

### Example 4

In order to test the effectiveness of a flexible sealing flange for air-tight closure of a test tube, the experiment of example 3 was repeated with antibiotic free milk only. For the controls, the aluminium foil of the Delvotest® SP test was completely punctured and removed and the incubation at 64 °C was carried out without covering the test tubes with adhesive foil as supplied and recommended by the manufacturer. After 2 hours and 45 minutes of incubation, the reaction zone was partly dried out. The nutrient tablet had absorbed part of the milk and another portion was evaporated. This drying out lead to a purple band in the upper part of the reaction zone, which could result in false positive test evaluation.

The use of a sample receiver containing the nutrients and connected to an operating means with a flexible sealing flange for air-tight closure of the test tube, did not lead to drying out of the sample reaction zone. After 2 hours and 45 minutes of incubation the sample reaction zone of the Delvotest was completely yellow, indicating that the milk was free of antibiotics.

## Claims

1. Test device for determining an analyte in a sample, the device comprising:
a reagent compartment (1) which is contained in a cylindrical housing (2), which cylindrical housing (2) comprises at least a first open end; and
a sample receiver (3) which is also contained in said cylindrical housing (2); and
an operating means (4) capable of moving said reagent compartment (1) and/or said sample receiver (3);
and wherein the reagent compartment (1) and the sample receiver
(3) can be in at least two positions relative to each other, said positions comprising a first position wherein the sample receiver (3) is not in analyte-exchangeable contact with the reagent compartment (1), and a second position wherein the sample receiver (3) is in analyte-exchangeable contact with the reagent compartment (1).

2. Test device according to claim 1, wherein the sample receiver (3) and the operating means are fixedly connected so as to form a sample taker (5).

3. Test device according to claim 2, wherein the sample taker (5) can be removed from the cylindrical housing.

4. Test device according to claim 1 or 2, wherein said cylindrical housing (2) comprises a second open end that can be closed by a removable lid (17), and wherein the sample receiver (3) is located between the reagent compartment (1) and the second open end.

5. Test device according to any one of the preceding claims, wherein said operating means (4) also comprises a closing means (7) for air-tight closure of said first open end.

6. Test device according to any one of the preceding claims, wherein said sample receiver (3) comprises absorbent materials, preferably said absorbent materials are mixed together and/or stacked in layers, said layers optionally being alternated or divided by one or more (semi)permeable membranes.

7. Test device according to claim 6, wherein the amount of absorbent materials provides defined sample volumes when soaked in sample fluid.

8. Test device according to any one of the preceding claims, wherein said sample receiver (3) comprises a probing element (15).

9. Test device according to according to any one of claims 4-8, wherein a sample uptake element (18) extends from said second open end.

10. Test device according to any one of the preceding claims, wherein said sample receiver (3) comprises reagents.

11. Method for determining the presence of an analyte in a sample comprising:
a) providing a test device for determining an analyte in a sample, the device comprising: a reagent compartment (1) which is contained in a cylindrical housing (2), which cylindrical housing (2) comprises at least a first open end; and a sample receiver (3) which is also contained in said cylindrical housing (2); and an operating means (4) capable of moving said reagent compartment (1) and/or said sample receiver (3); and wherein the reagent compartment (1) and the sample receiver (3) can be in at least two positions relative to each other, said positions comprising a first position wherein the sample receiver (3) is not in analyte-exchangeable contact with the reagent compartment (1), and a second position wherein the sample receiver (3) is in analyte-exchangeable contact with the reagent compartment (1);
b) bringing said sample receiver (3) in contact with the sample;
c) bringing the reagent compartment (1) and the sample receiver (3) in the second position thereby allowing exchange of analyte and/or reagents between the reagent compartment (1) and the sample receiver (3) and allowing the occurrence of a reaction between analyte and reagents and the formation of a detectable product; and
d) determining the presence of the analyte in the liquid sample by visual inspection of the reagent compartment (1) and/or the sample receiver (3) for the presence of the detectable product.

12. Method according to claim 11, wherein said sample receiver (3) is brought in contact with the sample by the use of a probing element (15) or sample uptake element (18) provided on the test device.

13. Use of test devices according to any one of the claims 1-10, for determining an analyte in a sample.

14. Use according to claim 13, wherein the sample is milk, liquor, serum, plasma and/or whole blood.

15. Use according to claim 13 or 14, wherein the analyte is a nucleotide, a peptide and/or a saccharide, and/or a polymer thereof.

16. Use according to any one of the claims 13 to 14, wherein the analyte is an a toxin, a pesticide, a antibiotic, an antibody, an antigen, an enzyme, a cofactor, a hormone, a virus, a bacterium and/or a fungus.

## Patentansprüche

1. Testvorrichtung zum Bestimmen eines Analyten in einer Probe, wobei die Vorrichtung
eine Reagenskammer (1), die in einem zylindrischen Gehäuse (2) enthalten ist, wobei das zylindrische Gehäuse (2) wenigstens ein erstes offenes Ende hat, und
eine Probenaufnahmeeinrichtung (3), die ebenfalls in dem zylindrischen Gehäuse (2) enthalten ist, und
eine Betätigungseinrichtung (4) aufweist, die in der Lage ist, die Reagenskammer (1) und/oder die Probenaufnahmeeinrichtung (3) zu bewegen,
und wobei die Reagenskammer (1) und die Probenaufnahmeeinrichtung (3) in wenigstens zwei Positionen relativ zueinander sein können, wobei die Positionen eine erste Position, in der die Probenaufnahmeeinrichtung (3) nicht in analytenaustauschbarem Kontakt mit der Reagenskammer (1) ist, und eine zweite Position umfassen, in der die Probenaufnahmeeinrichtung (3) in analytenaustauschbarem Kontakt mit der Reagenskammer (1) ist.

2. Testvorrichtung nach Anspruch 1, bei der die Probenaufnahmeeinrichtung (3) und die Betätigungseinrichtung fest verbunden sind, so dass sie eine Probennahmeeinrichtung (5) bilden.

3. Testvorrichtung nach Anspruch 2, bei der die Probennahmeeinrichtung (5) von dem zylindrischen Gehäuse entfernt werden kann.

4. Testvorrichtung nach Anspruch 1 oder 2, bei der das zylindrische Gehäuse (2) ein zweites offenes Ende aufweist, das durch einen abnehmbaren Deckel (17) verschlossen werden kann, und bei der die Probenaufnahmeeinrichtung (3) zwischen der Reagenskammer (1) und dem zweiten offenen Ende angeordnet ist.

5. Testvorrichtung nach einem der vorhergehenden Ansprüche, bei der die Betätigungseinrichtung (4) ferner ein Verschlussmittel (7) zum luftdichten Verschließen des ersten offenen Endes aufweist.

6. Testvorrichtung nach einem der vorhergehenden Ansprüche, bei der die Probenaufnahmeeinrichtung (3) absorbierende Materialien aufweist, wobei die absorbierenden Materialien bevorzugt miteinander vermischt und/oder in Schichten gestapelt sind, wobei sich die Schichten optional mit einer oder mehreren (semi-)permeablen Membranen abwechseln oder durch diese getrennt sind.

7. Testvorrichtung nach Anspruch 6, bei der die Menge absorbierender Materialien definierte Probenvolumina bereitstellt, wenn sie in Probenflüssigkeit getränkt sind.

8. Testvorrichtung nach einem der vorhergehenden Ansprüche, bei der die Probenaufnahmeeinrichtung (3) ein Sondenelement (15) aufweist.

9. Testvorrichtung nach einem der Ansprüche 4 bis 8, bei der sich ein Probennehmelement (18) von dem zweiten offenen Ende erstreckt.

10. Testvorrichtung nach einem der vorhergehenden Ansprüche, bei der die Probenaufnahmeeinrichtung (3) Reagenzien aufweist.

11. Verfahren zum Bestimmen der Anwesenheit eines Analyten in einer Probe mit den Schritten:
a) Bereitstellen einer Testvorrichtung zum Bestimmen eines Analyten in einer Probe, wobei die Vorrichtung eine Reagenskammer (1), die in einem zylindrischen Gehäuse (2) enthalten ist, wobei das zylindrische Gehäuse (2) wenigstens ein erstes offenes Ende hat, und eine Probenaufnahmeeinrichtung (3), die ebenfalls in dem zylindrischen Gehäuse (2) enthalten ist, und eine Betätigungseinrichtung (4) aufweist, die in der Lage ist, die Reagenskammer (1) und/oder die Probenaufnahmeeinrichtung (3) zu bewegen, und wobei die Reagenskammer (1) und die Probenaufnahmeeinrichtung (3) in wenigstens zwei Positionen relativ zueinander sein können, wobei die Positionen eine erste Position, in der die Probenaufnahmeeinrichtung (3) nicht in analytenaustauschbarem Kontakt mit der Reagenskammer (1) ist, und eine zweite Position umfassen, in der die Probenaufnahmeeinrichtung (3) in analytenaustauschbarem Kontakt mit der Reagenskammer (1) ist,
b) Inkontaktbringen der Probenaufnahmeeinrichtung (3) mit der Probe,
c) Bringen der Reagenskammer (1) und der Probenaufnahmeeinrichtung (3) in die zweite Position, wodurch ein Austausch des Analyten und/oder von Reagenzien zwischen der Reagenskammer (1) und der Probenaufnahmeeinrichtung (3) ermöglicht wird und das Auftreten einer Reaktion zwischen Analyt und Reagenzien und die Bildung eines detektierbaren Produkts ermöglicht wird, und
d) Bestimmen der Anwesenheit des Analyten in der flüssigen Probe durch visuelle Prüfung der Reagenskammer (1) und/oder der Probenaufnahmeeinrichtung (3) in Bezug auf die Anwesenheit des detektierbaren Produkts.

12. Verfahren nach Anspruch 11, bei dem die Probenaufnahmeeinrichtung (3) durch die Verwendung eines an der Testvorrichtung vorgesehenen Sondenelements (15) oder eines an der Testvorrichtung vorgesehenen Probennehmelements (18) in Kontakt mit der Probe gebracht wird.

13. Verwendung der Testvorrichtung nach einem der Ansprüche 1 bis 10 zur Bestimmung eines Analyten in einer Probe.

14. Verwendung nach Anspruch 13, bei der die Probe Milch, Körperflüssigkeit, Serum, Plasma und/oder Vollblut ist.

15. Verwendung nach Anspruch 13 oder 14, bei der das Analyt ein Nukleotid, ein Peptid und/oder ein Saccharid und/oder ein Polymer davon ist.

16. Verwendung nach einem der Ansprüche 13 bis 14, bei der das Analyt ein Toxin, ein Pestizid, ein Antibiotikum, ein Antikörper, ein Antigen, ein Enzym, ein Co-Faktor, ein Hormon, ein Virus, ein Bakterium und/oder ein Pilz ist.

## Revendications

1. Dispositif de test pour déterminer un analyte dans un échantillon, le dispositif comportant :
un compartiment de réactif (1) qui est contenu dans un boîtier cylindrique (2), lequel boîtier cylindrique (2) comporte au moins une première extrémité ouverte, et
un récepteur d'échantillon (3) qui est également contenu dans ledit boitier cylindrique (2), et
des moyens d'actionnement (4) capables de déplacer ledit compartiment de réactif (1) et/ou ledit récepteur d'échantillon (3),
**caractérisé en ce que** le compartiment de réactif (1) et le récepteur d'échantillon (3) peuvent être dans au moins deux positions l'un par rapport à l'autre, lesdites positions comportant une première position dans laquelle le récepteur d'échantillon (3) n'est pas dans un contact d'échange d'un analyte possible avec le compartiment de réactif (1), et une seconde position dans laquelle le récepteur d'échantillon (3) est dans un contact d'échange d'analyte possible avec le compartiment de réactif (1).

2. Dispositif de test selon la revendication 1, dans lequel le récepteur d'échantillon (3) et les moyens d'actionnement sont reliés de manière fixe pour former un dispositif de prélèvement d'échantillon (5).

3. Dispositif de test selon la revendication 2, dans lequel le dispositif de prélèvement d'échantillon (5) peut être enlevé du boîtier cylindrique.

4. Dispositif de test selon la revendication 1 ou 2, dans lequel ledit boîtier cylindrique (2) comporte une seconde extrémité ouverte qui peut être fermée par un couvercle amovible (17), et dans lequel le récepteur d'échantillon (3) est positionné entre le compartiment de réactif (1) et la seconde extrémité ouverte.

5. Dispositif de test selon l'une quelconque des revendications précédentes, dans lequel lesdits moyens d'actionnement (4) comportent également des moyens de fermeture (7) pour une fermeture étanche à l'air de ladite première extrémité ouverte.

6. Dispositif de test selon l'une quelconque des revendications précédentes, dans lequel ledit récepteur d'échantillon (3) comporte des matériaux absorbants, de préférence lesdits matériaux absorbants sont mélangés ensemble et/ou empilés en couches, lesdites couches étant facultativement alternées ou divisées par une ou plusieurs membranes (semi)perméables.

7. Dispositif de test selon la revendication 6, dans lequel la quantité de matériaux absorbants fournit des volumes d'échantillon définis lorsqu'ils sont trempés dans un fluide échantillon.

8. Dispositif de test selon l'une quelconque des revendications précédentes, dans lequel ledit récepteur d'échantillon (3) comporte un élément de sondage (15).

9. Dispositif de test selon l'une quelconque des revendic4 à 8, dans lequel un élément de captage d'échantillon (18) s'étend depuis ladite seconde extrémité ouverte.

10. Dispositif de test selon l'une quelconque des revendications précédentes, dans lequel ledit récepteur d'échantillon (3) comporte des réactifs.

11. Procédé pour déterminer la présence d'un analyte dans un échantillon, comportant les étapes consistant à :
a) fournir un dispositif de test pour déterminer un analyte dans un échantillon, le dispositif comportant : un compartiment de réactif (1) qui est contenu dans un boîtier cylindrique (2), lequel boîtier cylindrique (2) comporte au moins une première extrémité ouverte, et un récepteur d'échantillon (3) qui est également contenu dans ledit boitier cylindrique (2), et des moyens d'actionnement (4) capables de déplacer ledit compartiment de réactif (1) et/ou ledit récepteur d'échantillon (3), et dans lequel le compartiment de réactif (1) et le récepteur d'échantillon (3) peuvent être dans au moins deux positions l'un par rapport à l'autre, lesdites positions comportant une première position dans laquelle le récepteur d'échantillon (3) n'est pas en contact d'échange d'analyte possible avec le compartiment de réactif (1), et une seconde position dans laquelle le récepteur d'échantillon (3) est dans un contact d'échange d'analyte possible avec le compartiment de réactif (1),
b) amener ledit récepteur d'échantillon (3) en contact avec l'échantillon,
c) amener le compartiment de réactif (1) et le récepteur d'échantillon (3) dans la seconde position, en permettant ainsi un échange d'analyte et/ou de réactifs entre le compartiment de réactif (1) et le récepteur d'échantillon (3), et en permettant l'apparition d'une réaction entre l'analyte et les réactifs, et la formation d'un produit détectable, et
d) déterminer la présence de l'analyte dans l'échantillon liquide par une inspection visuelle du compartiment de réactif (1) et/ou du récepteur d'échantillon (3) concernant la présence du produit détectable.

12. Procédé selon la revendication 11, dans lequel ledit récepteur d'échantillon (3) est amené en contact avec l'échantillon par l'intermédiaire de l'utilisation d'un élément de sondage (15) ou d'un élément de captage d'échantillon (18) agencé sur le dispositif de test.

13. Utilisation de dispositifs de test selon l'une quelconque des revendications 1 à 10, pour déterminer un analyte dans un échantillon.

14. Utilisation selon la revendication 13, dans laquelle l'échantillon est du lait, une liqueur, du sérum, du plasma et/ou du sang complet.

15. Utilisation selon la revendication 13 ou 14, dans laquelle l'analyte est un nucléotide, un peptide et/ou un saccharide, et/ou un polymère de ceux-ci.

16. Utilisation selon l'une quelconque des revendications 13 et 14, dans laquelle l'analyte est une toxine, un pesticide, un antibiotique, un anticorps, un antigène, une enzyme, un cofacteur, une hormone, un virus, une bactérie et/ou un champignon.
